# EUROPÄISCHE PATENTSCHRIFT

(11) **EP 3 310 768 B1**
(45) Veröffentlichungstag und Bekanntmachung des Hinweises auf die Patenterteilung: **15.04.2020**
(21) Anmeldenummer: 16730815.4
(22) Anmeldetag: 17.06.2016
(51) Int. Cl.: C07D 307/33

(54) **HERSTELLUNG VON PANTOLACTON**
PREPARATION OF PANTOLACTONE
PRÉPARATION DE LA PANTOLACTONE

(30) Priorität: 19.06.2015 EP 15172900
(43) Veröffentlichungstag der Anmeldung: 25.04.2018
(73) Patentinhaber: BASF SE, 67056 Ludwigshafen am Rhein (DE)
(72) Erfinder: FISCHER, Klaus, 67434 Neustadt (DE); NEHLS, Benjamin, 68165 Mannheim (DE); DESCHLER, Juergen, 67471 Elmstein (DE); DOBLER, Walter, 68723 Schwetzingen (DE); LAUTERBACH, Arnulf, 67067 Ludwigshafen (DE); SCHLAUTMANN, Sabine, 67067 Ludwigshafen (DE)
(74) Vertreter: BASF IP Association
(86) Internationale Anmeldenummer: PCT/EP2016/063978
(87) Internationale Veröffentlichungsnummer: WO 2016/202965

(56) Entgegenhaltungen:
- GB-A- 597 648
- ROWICKI T ET AL: "Calcium pantothenate. Part 1. (R,S)-pantolactone technology improvement at the tonnage scale", INDUSTRIAL & ENGINEERING CHEMISTRY RESEARCH, AMERICAN CHEMICAL SOCIETY, US, Bd. 45, Nr. 4, 15. Februar 2006 (2006-02-15), Seiten 1259-1265, XP002505647, ISSN: 0888-5885, DOI: 10.1021/IE050774U [gefunden am 2006-01-21]
- HAUGHTON L ET AL: "Enzymatic kinetic resolution of pantolactone:relevance to chiral auxiliary chemistry", TETRAHEDRON ASYMMETRY, PERGAMON PRESS LTD, OXFORD, GB, Bd. 11, Nr. 8, 1. Mai 2000 (2000-05-01), Seiten 1697-1701, XP004204572, ISSN: 0957-4166, DOI: 10.1016/S0957-4166(00)00130-0

## Beschreibung

### HINTERGRUND DER ERFINDUNG

Die vorliegende Erfindung betrifft ein neues Verfahren zur Herstellung von Pantolacton durch Umsetzung von Hydroxypivalaldehydcyanhydrin in einem Phasentrennverfahren.

### STAND DER TECHNIK

Pantolacton (α-Hydroxy-β,β-dimethyl-γ-butyrolacton, Dihydro-3-hydroxy-4,4-dimethyl-2(3*H*-furanon) ist eine wertvolle Verbindung für die Herstellung kosmetischer und pharmazeutischer Produkte. So dient D-Pantolacton als Ausgangsmaterial für die Herstellung von Panthenol, auch als Dexpanthenol oder Pantothenol bezeichnet. Panthenol wird seit langem als Wirkstoff zur topischen Anwendung bei Erkrankungen der Haut und Schleimhäute und kosmetisch in Hautpflegemitteln verwendet. Pantothensäure (Vitamin B₅), das Umsetzungsprodukt von Pantolacton mit β-Alanin, ist erforderlich für den Aufbau von Coenzym A, das im Stoffwechsel den Transfer von Acylgruppen katalysiert. Es ist beteiligt am Auf- und Abbau von Kohlenhydraten, Fetten, Aminosäuren und an der Synthese von Cholesterin, welches im Organismus für die Bildung der Steroidhormone gebraucht wird.

Es ist bekannt, dass man Formaldehyd mit Isobutyraldehyd in einer Aldoladdition zum Hydroxypivalaldehyd (α,α-Dimethyl-β-hydroxypropionaldehyd) umsetzen kann und diesen Aldehyd dann weiter durch Addition von Blausäure zum Hydroxypivalaldehydcyanhydrin (2,4-Dihydroxy-3,3-dimethylbutyronitril) umwandeln kann, das als Ausgangsverbindung zur Herstellung von Pantolacton bzw. den durch Verseifung des Pantolactons resultierenden Pantoaten dient.

Die US 2,328,000 beschreibt die Herstellung von racemischem Pantolacton durch Umsetzung von Formaldehyd und Isobutyraldehyd in Gegenwart eines Alkalimetallcarbonats zum Hydroxypivalaldehyd und dann weiter durch Addition von Blausäure zum Cyanhydrin. Das Cyanhydrin wird dann mit konzentrierter HCl zum Lacton umgesetzt. Dabei wird in dem einzigen Ausführungsbeispiel eine etherische Lösung des Cyanhydrins mit konzentrierter HCl versetzt, die Mischung über Nacht stehen gelassen, wobei Ammoniumchlorid auskristallisiert, nach Wasserzugabe der Ether abdestilliert, die resultierende Mischung mit NaOH auf pH 7,2 eingestellt und dann kontinuierlich mit Ether extrahiert. Aus den vereinigten Etherextrakten wird das Pantolacton isoliert.

Die FR 1 175 516 beschreibt ebenfalls ein Verfahren zur Herstellung von Pantolacton, ausgehend von Formaldehyd und Isobutyraldehyd durch Aldolreaktion, Bildung des Cyanhydrins und dessen Hydrolyse unter Ringschluss. Das Pantolacton wird durch Extraktion isoliert und destillativ gereinigt.

Die DE-OS-26 27 940 beschreibt ein Verfahren zur Herstellung von racemischem Pantolacton, bei dem in einer ersten Stufe Hydroxypivalaldehyd mit in situ hergestellter Cyanwasserstoffsäure umgesetzt und anschließend in einer zweiten Stufe das dabei erhaltene Cyanhydrin in stark saurem Milieu hydrolysiert und das gebildete Pantolacton abgetrennt und gereinigt wird, wobei a) in beiden Verfahrensstufen als einziges Lösungsmittel ein Alkohol mit 5 oder 6 C-Atomen verwendet wird, b) in der ersten Stufe die Umsetzung des Hydroxypivalaldehyds mit HCl und einem Alkalicyanid bei 10 bis 20 °C und einem pH-Wert von 8,2 bis 9,2 erfolgt, wobei ein 10%iger HCN-Überschuss eingesetzt wird und c) in der zweiten Stufe die saure Hydrolyse mit überschüssiger konzentrierter HCl bei einer Temperatur von 100 bis 105 °C über einen Zeitraum von 20 bis 30 Minuten durchgeführt und anschließend das Pantolacton abgetrennt wird.

Die DE-OS-27 58 883 beschreibt ein Verfahren zur Herstellung von Pantolacton, bei dem man Formaldehyd und Isobutyraldehyd in Gegenwart von 0,01 bis 0,3 Mol tertiärem Amin je Mol Isobutyraldehyd umsetzt, das so gebildete Reaktionsgemisch mit Blausäure umsetzt, wobei die Blausäurekonzentration während der Reaktion nicht mehr als ein Gewichtsprozent bezogen auf das Reaktionsgemisch beträgt und das so erhaltene Reaktionsgemisch mit gasförmigem Chlorwasserstoff umsetzt. Zur Aufarbeitung des Endprodukts wird eine Extraktion, z. B. mit Methylenchlorid und Destillation des Lösungsmittels beschrieben.

Es ist bekannt, zur Herstellung von enantiomerenangereichertem Pantolacton die Cyanhydrinsynthese enantioselektiv durchzuführen. So beschreiben F. Effenberger et al. in Tetrahedron Asymmetry Vol. 6, No. 1, Seiten 271 - 282, 1995 die enzymkatalysierte Addition von Blausäure an substituierte Pivalaldehyde zur Herstellung von (R)-Pantolacton.

L. Synoradzki et al. beschreiben in Organic Research & Development 2006, 10, 103 - 108 eine verbesserte Oxynitrilase katalysierte asymmetrische Hydrocyanierung von Hydroxypivalaldehyd zur Herstellung von (R)-Pantolacton. Dabei kommt eine Oxynitrilase aus Mandel-, Apfel- und Pflaumenkernen zum Einsatz.

Die EP 0 528 256 A1 beschreibt die enzymatische Herstellung von D-2,4-Dihydroxy-3,3-dimethylbutyronitril als chirale Zwischenstufe von D-Pantolacton und D-Pantothensäure durch Umsetzung von Hydroxypivalaldehyd mit Cyanwasserstoff in Gegenwart von D-Oxynitrilase. Die Umsetzung des Cyanhydrins zum Pantolacton erfolgt in einem wässrig-sauren Medium bei erhöhter Temperatur. Als geeignete Säuren werden Mineralsäuren, wie Schwefelsäure oder konzentrierte Salzsäure, genannt. Zur Isolierung des Pantolactons wird das Reaktionsgemisch mit einem organischen Lösungsmittel extrahiert.

T. Rowicki et al. beschreiben in Ind. Eng. Chem. Res. 2006, 45, 1259-1265 ein Verfahren zur Herstellung von Pantolacton durch Umsetzung von Formaldehyd und Isobutyraldehyd zu Hydroxypivalaldehyd, welcher anschließend mit NaCN zu Hydroxypivalaldehydcyanhydrin umgesetzt wird. Konkret wird eine Lösung des Cyanhydrins in einem Methanol/Wasser-Gemisch mit 50%iger Schwefelsäure versetzt, auf 100°C erhitzt und Methanol abdestilliert. Nach dem Abkühlen erhält man ein zweiphasiges Gemisch, welches durch Zugabe von Na₂CO₃ auf einen pH-Wert von 6 eingestellt wird. Nach Zugabe von Dichlormethan und rühren wird das Produkt von der wässrigen Phase abgetrennt.

Die bekannten Verfahren zur Herstellung von Pantolacton weisen verschiedene Nachteile auf. So sind Verfahren, bei denen als Endprodukt eine wässrige Pantoat-Lösung mit geringer Wertstoffkonzentration (häufig unter 30 %) erhalten wird, bezüglich der erzielbaren Produktionskapazität beschränkt. Zudem ist eine Aufkonzentration von wässrigen Produktlösungen immer sehr energieaufwändig. Verfahren, nach denen das Pantolacton durch Extraktion mit einem organischen Lösungsmittel isoliert wird, erfordern Maßnahmen zur Reinigung und Wiederverwertung des Lösungsmittels. Der Einsatz von Chlorwasserstoff und speziell von gasförmigem HCl zur Überführung des Cyanhydrins in das Pantolacton ist aufgrund der hohen Korrosivität nicht unproblematisch. Wird dann zur Neutralisation NaOH eingesetzt, so weisen die erhaltenen Produkte in der Regel einen hohen Natriumchloridgehalt auf, der sich nachteilig auf die folgende Racematspaltung auswirken kann. Der Einsatz von HCl und NaOH führt zudem zu höheren Gestehungskosten. Zudem wird beim Einsatz von Alkalimetallhydroxiden das Pantolacton in der Regel zu den entsprechenden Pantoaten verseift. Die Racematspaltung der Pantoate ist jedoch komplex und kostenintensiv. Daher ist es von Vorteil, wenn als Produkt Pantolacton als Enantiomerengemisch erhalten wird, das dann einer enzymatischen Enantiomerentrennung unterzogen werden kann.

Der vorliegenden Erfindung liegt die Aufgabe zugrunde, ein verbessertes Verfahren zur Herstellung von Pantolacton zur Verfügung zu stellen, das möglichst viele der zuvor genannten Nachteile überwindet.

Überraschenderweise wurde jetzt gefunden, dass man Pantolacton vorteilhaft herstellen kann, wenn man Hydroxypivalaldehydcyanhydrin zunächst mit einem ausreichenden molaren Überschuss von Neutralisationsäquivalenten einer Säure in einem einphasigen Reaktionsgemisch umsetzt und dieses anschließend einer Neutralisation unterzieht, wobei vermieden wird, dass das nach Abschluss der Neutralisation erhaltene Reaktionsgemisch einen zu hohen pH-Wert und einen zu hohen Wassergehalt aufweist. Vorteilhafterweise trennt sich dabei während der Neutralisation das Reaktionsgemisch in zwei Phasen, wobei die obere organische Phase in hoher Konzentration das Pantolacton enthält und die untere wässrige Phase eine hoch konzentrierte, vorzugsweise gesättigte, Salzlösung enthält.

### ZUSAMMENFASSUNG DER ERFINDUNG

Gegenstand der Erfindung ist ein Verfahren zur Herstellung von Pantolacton (I) 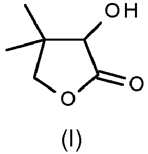 bei dem man
a) eine wässrige Zusammensetzung, die Hydroxypivalaldehydcyanhydrin (II) 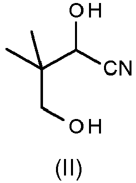 enthält, als Ausgangsmaterial bereitstellt,
b) das in Schritt a) bereitgestellte Ausgangsmaterial einer Umsetzung mit H₂SO₄ als Säure bei einem pH-Wert von kleiner als 1,1 in einer homogenen flüssigen Phase unterzieht,
c) dem Umsetzungsprodukt aus Schritt b)NH₃ in gasförmiger oder wässriger Form als Base zugegeben wird, bis der pH-Wert in einem Bereich von 3 bis 7 liegt und wobei der Wassergehalt des Reaktionsgemischs in Schritt c) nach Zugabe der Base in einem Bereich von 40 bis 60 Gew.-%, bezogen auf das Gesamtgewicht des Reaktionsgemischs, liegt, wobei sich eine organische Phase bildet, die das Pantolacton (I) enthält, und eine wässrige Phase, die das Basensalz der starken Säure enthält,
d) die Phasen separieren lässt und die organische Phase von der wässrigen Phase abtrennt.

### BESCHREIBUNG DER ERFINDUNG

Das erfindungsgemäße Verfahren weist die folgenden Vorteile auf:
- Das Produkt lässt sich nach dem erfindungsgemäßen Phasentrennverfahren als organische Phase (obere Phase) mit sehr hohem Pantolactongehalt isolieren. Als wässrige Phase (untere Phase) wird eine hoch konzentrierte, vorzugsweise gesättigte, Salzlösung erhalten (beim Einsatz von H₂SO₄ als Säure und von NH₃ als Base beispielsweise eine gesättigte wässrige Ammoniumsulfatlösung).
- Auf den Einsatz von externen organischen Lösungsmitteln, z. B. als Extraktionsmittel, kann verzichtet werden.
- In dem erfindungsgemäßen Verfahren kann auf den Einsatz von Chlorwasserstoff und speziell von gasförmigem HCl zur Überführung des Cyanhydrins in das Pantolacton verzichtet werden. Es kann auch auf den Einsatz von Alkalimetallhydroxiden zur Neutralisation verzichtet werden. Das erfindungsgemäße Verfahren ermöglicht somit die Herstellung von Pantolacton mit geringem Natriumchloridgehalt.
- Mit dem erfindungsgemäßen Phasentrennverfahren gelingt es, die Verseifung des Pantolactons zum Pantoat zu vermeiden. Das so in Form eines D,L-Isomerengemischs erhaltene Pantolacton eignet sich vorteilhaft für den Einsatz in einer enzymatischen Enantiomerentrennung.

Zur Bestimmung der pH-Werte können im erfindungsgemäßen Verfahren handelsübliche pH-Wert-Messketten auf Basis einer pH-Elektrode und einer Referenzelektrode, beispielsweise in Form einer Einstabmesskette, eingesetzt werden. Sofern im Folgenden nichts anderes angegeben ist, sind die pH-Werte temperaturkompensiert. Zur Kompensation kann beispielsweise eine pH-Wert-Messkette eingesetzt werden, die über einen Temperaturfühler, z. B. ein Widerstandsthermometer, verfügt. Derartige Messketten ermitteln die Temperatur am Messort und geben diese an die Messeinrichtung weiter, so dass die Temperaturkorrektur automatisch erfolgt. Wenn die Messlösung keinen oder nur geringen Temperaturschwankungen unterliegt, ist es auch möglich, die Temperatur separat einmal oder in ausreichenden Zeitabständen zu ermitteln und den so ermittelten Wert manuell an der Messeinrichtung einzugeben, die dann auf Basis dieser Temperaturangabe die pH-Wertkorrektur vornimmt. Die pH-Wertbestimmung in dem zweiphasigen Reaktionsgemisch in Schritt c) erfolgt vorzugsweise in der gut durchmischten Emulsion. Es hat sich gezeigt, dass die so ermittelten Messwerte den nach einer Phasentrennung in der wässrigen Phase ermittelten Werten entsprechen.

Im Rahmen der Erfindung wird ein Reaktionssystem als homogen bezeichnet, wenn es aus einer einzigen Phase besteht und als heterogen, wenn es aus mehreren Phasen aufgebaut ist. Eine Umsetzung in einer homogenen flüssigen Phase ist gleichbedeutend mit einer einphasigen Umsetzung in flüssiger Phase.

Sofern im Folgenden nicht genauer angegeben, bezeichnet "Pantolacton" und die Formel (1) sowohl die Enantiomere in Reinform als auch racemische und optisch aktive Gemische der Enantiomeren des Pantolactons. Lediglich zur Veranschaulichung werden im Folgenden D-Pantolacton und L-Pantolacton wiedergegeben:

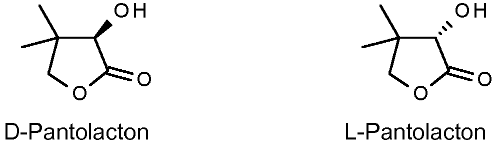

Die Begriffe "Hydroxypivalaldehyd" und "Hydroxypivalinaldehyd" werden synonym verwendet.

### Schritt a)

In Schritt a) des erfindungsgemäßen Verfahrens wird eine wässrige Zusammensetzung bereitgestellt, die Hydroxypivalaldehydcyanhydrin (II) enthält. Die Bereitstellung kann nach dem Fachmann bekannten Verfahren erfolgen.

Vorzugsweise wird zur Bereitstellung des Ausgangsmaterials in Schritt a):
a1) Formaldehyd mit Isobutyraldehyd einer Aldoladdition unter Erhalt von Hydroxypivalaldehyd unterzogen, und
a2) der Hydroxypivalaldehyd mit einer Cyanidquelle unter Erhalt von Hydroxypivalaldehydcyanhydrin (II) umgesetzt.

Ein solches Verfahren ist dem Fachmann bekannt und beispielsweise in der DE-OS-27 58 883 beschrieben, worauf hier Bezug genommen wird.

Formaldehyd und Isobutyraldehyd stehen in großtechnischem Maßstab zur Verfügung.

Die Umsetzung in Schritt a1) erfolgt vorzugsweise bei einer Temperatur von 20 bis 105 °C, vorzugsweise von 40 bis 95 °C.

Die Umsetzung in Schritt a1) kann prinzipiell bei Unterdruck, Normaldruck oder Überdruck durchgeführt werden.

Man kann die Aldehyde in stöchiometrischer Menge oder jeden der Aldehyde im Überschuss einsetzen. Vorzugsweise wird der Formaldehyd in einer Menge von 0,9 bis 1,5 Mol, insbesondere 0,9 bis 1,1 Mol, je Mol Isobutyraldehyd eingesetzt.

In einer speziellen Ausführungsform wird das Reaktionsgemisch aus Schritt a1) ohne Aufarbeitung im Schritt a2) eingesetzt.

Im Schritt a2) wird als Cyanidquelle vorzugsweise Blausäure (HCN) eingesetzt. Die Blausäure kann als Gas oder zweckmäßigerweise in Form einer wässrigen Lösung verwendet werden.

Die Umsetzung in Schritt a2) erfolgt vorzugsweise bei einer Temperatur von 10 bis 60 °C, besonders bevorzugt von 15 bis 50 °C.

Die Umsetzung in Schritt a2) kann prinzipiell bei Unterdruck, Normaldruck oder Überdruck durchgeführt werden. Bevorzugt wird die Reaktion unter Umgebungsdruck durchgeführt.

### Schritt b)

Erfindungsgemäß wird in Schritt b) H₂SO₄ als Säure eingesetzt.

In einer speziellen Ausführungsform wird als Säure in Schritt b) wenigstens 90%ige H₂SO₄, eingesetzt wird. Geeignet ist beispielsweise eine 96%ige H₂SO₄, wie sie großtechnisch zur Verfügung steht.

Bevorzugt liegt in Schritt b) das Molverhältnis der eingesetzten Säure zu Hydroxypivalaldehydcyanhydrin (II) in einem Bereich von 0,5 : 1 bis 2 : 1 bevorzugt 0,7 : 1 bis 1,5 : 1.

In einer speziellen Ausführungsform wird in Schritt b) das Hydroxypivalaldehydcyanhydrin-haltige Ausgangsmaterial einer Umsetzung mit 0,9 bis 1,2 Mol-Äquivalenten H₂SO₄, bevorzugt 1,05 bis 1,10 Mol-Äquivalenten H₂SO₄, bezogen auf Hydroxypivalaldehydcyanhydrin unterzogen.

Bevorzugt wird während der Umsetzung in Schritt b) der pH-Wert des Reaktionsgemischs in einem Bereich von 0 bis 1,2, bevorzugt von 0,2 bis 1, gehalten.

Bevorzugt liegt während der Umsetzung in Schritt b) ein einphasiges Reaktionsgemisch vor.

Bevorzugt erfolgt die Umsetzung in Schritt b) bei einer Temperatur von 50 bis 110 °C, besonders bevorzugt von 60 bis 105 °C, insbesondere von 75 bis 100 °C.

In einer ersten bevorzugten Ausführungsform erfolgt die Umsetzung in Schritt b) bei einem Druck im Bereich von 850 bis 1150 mbar, bevorzugt von 900 bis 1100 mbar. Diese Normaldruckvariante ist vorteilhaft, da auf aufwändige Vorrichtungen für ein Arbeiten unter vermindertem oder erhöhtem Druck verzichtet werden kann. Sie kommt speziell dann zum Einsatz, wenn während der Umsetzung in Schritt b) keine flüchtigen Komponenten, wie z. B. Wasser, aus dem Reaktionsgemisch entfernt werden sollen.

In einer zweiten bevorzugten Ausführungsform erfolgt die Umsetzung in Schritt b) bei einem Druck im Bereich von 100 bis 800 mbar, bevorzugt von 200 bis 700 mbar. Diese Niederdruckvariante kommt speziell dann zum Einsatz, wenn während der Umsetzung in Schritt b) flüchtige Komponenten, speziell Wasser, aus dem Reaktionsgemisch entfernt werden sollen. In einer bevorzugten Ausführungsform der Erfindung wird dementsprechend während der Umsetzung in Schritt b) destillativ Wasser aus dem Reaktionsgemisch entfernt. Dies ermöglicht es, bei der Neutralisation im folgenden Schritt c) wässrige Basen einzusetzen, ohne den Wassergehalt dabei unerwünscht zu erhöhen. So wird das Ziel, dass die in Schritt c) erhaltene wässrige Phase das Basensalz der starken Säure in möglichst konzentrierter Form enthält, erreicht. Beim Einsatz von NH₃ als Base ist es somit beispielsweise möglich, anstelle von NH₃-Gas wässrigen Ammoniak einzusetzen, was eine geringere Wärmetönung zur Folge hat.

In einer speziellen Variante der zuvor beschriebenen zweiten bevorzugten Ausführungsform erfolgt die Umsetzung in Schritt b) bei einem Druck im Bereich von 100 bis 800 mbar, bevorzugt von 200 bis 700 mbar, und wird während der Umsetzung in Schritt b) destillativ Wasser aus dem Reaktionsgemisch entfernt und wird in Schritt c) dem Reaktionsprodukt aus Schritt b) wässriger Ammoniak als Base zugegeben.

Die Umsetzung in Schritt b) kann in Gegenwart eines Inertgases, wie Stickstoff, Neon oder Argon, erfolgen.

Die Umsetzung in Schritt b) kann diskontinuierlich oder kontinuierlich durchgeführt werden. Vorzugsweise wird die Umsetzung in Schritt b) kontinuierlich durchgeführt.

Geeignete Reaktoren für die Umsetzung in Schritt b) sind prinzipiell Reaktoren, die zur Durchführung von chemischen Umsetzungen in flüssiger Phase geeignet sind. Für die Umsetzung in Schritt b) kann ein Reaktor oder können mehrere, gleiche oder verschiedene Reaktoren eingesetzt werden. Im einfachsten Fall erfolgt die Umsetzung in Schritt b) in einem einzelnen Reaktor. Werden mehrere Reaktoren eingesetzt, so können diese jeweils gleiche oder verschiedene Vermischungscharakteristiken aufweisen. Die einzelnen Reaktoren können, falls gewünscht, durch Einbauten ein- oder mehrfach unterteilt sein. Werden zwei oder mehr als zwei (z. B. 3, 4 oder 5) Reaktoren eingesetzt, so können diese untereinander beliebig verschaltet sein, z. B. parallel oder hintereinander (in Reihe). In einer bevorzugten Ausführung wird ein einzelner Reaktor oder wird eine Kaskade aus wenigstens zwei in Reihe geschalteten Reaktoren eingesetzt.

Geeignete Reaktoren sind z. B. Rohrreaktoren, die gegebenenfalls mit Einbauten versehen sein können, Rührkessel, Schlaufenreaktoren, etc. Geeignete Schlaufenreaktoren sind z. B. Freistrahlreaktoren, Strahlschlaufenreaktoren, Strahldüsenreaktoren, etc. Zur Durchmischung der Reaktoren können übliche Mischvorrichtungen eingesetzt werden. Dazu zählen z. B. statische oder dynamische Mischer, Vorrichtungen zum Umpumpen des Reaktionsgemischs und Kombinationen davon. Bevorzugt wird wenigstens eine Mischvorrichtung eingesetzt, die ausgewählt ist unter Rührern, Mischdüsen, Mischpumpen, statischen Mischelementen, Füllkörperschüttungen, etc. Geeignete Rührertypen umfassen z. B. Blattrührer, Ankerrührer, Schrägblattrührer, Balkenrührer, Wendelrührer, Schneckenrührer, Propellerrührer, Impellerrührer, Scheibenrührer, Turbinenrührer, etc.

Die Reaktoren können mit üblichen Vorrichtungen zur Zuführung oder Abführung von Wärme ausgestattet sein. Geeignete Wärmeaustauscher sind die üblichen, dem Fachmann bekannten Vorrichtungen, in welchen Wärme von einem Medium auf ein anderes übertragen wird, wie z. B. Rohrschlangen-, Platten-, Ringnut-, Rippenrohr-, Lamellen-, Doppelrohr-, Rohrbündel-, Spaltrohr-, Teller-, Kerzen-, Spiral-, Block-, Schnecken- und Wendelwärmeaustauscher.

Bevorzugt wird für die Umsetzung in Schritt b) ein einzelner Rührkessel oder eine Rührkesselkaskade aus zwei oder drei Rührkesseln eingesetzt.

Die zur Umsetzung in Schritt b) eingesetzten Reaktoren können über eine Vorrichtung zur Zuführung eines Inertgases verfügen. Die Zuführung von Inertgasen kann dabei in die im Reaktor befindliche flüssige Phase oder in die Gasphase erfolgen.

Im einfachsten Fall erfolgt die Umsetzung in Schritt b) in batch-Fahrweise in einem einzelnen Reaktor, vorzugsweise einem Rührkessel. Dann kann beispielsweise eine wässrige Lösung des Cyanhydrins in dem Reaktor vorgelegt und die starke Säure zugegeben werden. Vorzugsweise erfolgt die Säurezugabe unter guter Durchmischung des Reaktionsgemischs. Aufgrund der Verdünnungswärme erfolgt bereits durch die Säurezugabe eine Erwärmung des Reaktionsgemischs. Zusätzlich wird der flüssige Reaktorinhalt erwärmt, um die zur Umsetzung gewünschte Reaktionstemperatur zu erreichen. Mit der Erwärmung kann in der Regel begonnen werden, wenn der Reaktor eine ausreichende Menge Flüssigkeit enthält, die eine Durchmischung des Reaktorinhalts erlaubt. Wird die Reaktion in Schritt b) in batch-Fahrweise durchgeführt, so liegt die Reaktionsdauer vorzugsweise in einem Bereich von 30 Minuten bis 600 Minuten, vorzugsweise 45 Minuten bis 300 Minuten, insbesondere 60 Minuten bis 240 Minuten, wie z. B. 120 Minuten. Als Reaktionsdauer wird dabei die Zeit verstanden, ab der das Reaktionsgemisch erstmals auf die Mindestreaktionstemperatur erwärmt ist bis zum Abschluss der Reaktion.

Die Umsetzung in Schritt b) wird bevorzugt kontinuierlich durchgeführt. Dann kann die Umsetzung in einem einzelnen Reaktor oder in einem Reaktionssystem aus mehreren Reaktoren erfolgen. In einer speziellen Ausführung des erfindungsgemäßen Verfahrens erfolgt die Umsetzung in Schritt b) in mehreren (z. B. zwei, drei, vier oder mehr als vier) Reaktoren. Diese sind vorzugsweise alle in Reihe geschaltet. Bevorzugt erfolgt die Umsetzung in zwei oder drei, insbesondere in zwei in Reihe geschalteten Reaktoren. Als erster Reaktor wird vorzugsweise ein Rührreaktor eingesetzt, der über zwei separate Zuläufe für das Hydroxypivalaldehydcyanhydrin-haltige Ausgangsmaterial und die starke Säure verfügt. Gewünschtenfalls können weitere Zuläufe vorhanden sein, z. B. um dem Reaktionsgemisch Wasser oder andere Lösungsmittel zuzugeben. Eine solche Zugabe von Wasser oder anderen Lösungsmitteln ist jedoch in der Regel nicht erforderlich. Zum Anfahren der Reaktion kann der erste Reaktor parallel mit dem Hydroxypivalaldehydcyanhydrin-haltigen Ausgangsmaterial und der starken Säure befüllt werden. Vorzugsweise erfolgt das Anfahren unter guter Durchmischung des Reaktionsgemischs. Dazu kann es sinnvoll sein, den ersten Reaktor zunächst mit einer Mindestmenge des Hydroxypivalaldehydcyanhydrin-haltigen Ausgangsmaterials zu befüllen, bis eine Durchmischung des Reaktorinhalts möglich ist, und erst dann mit der Zuführung der starken Säure zu beginnen. Bei der kontinuierlichen Durchführung der Umsetzung in Schritt b) wird mit der Erwärmung des Reaktionsgemischs in der Regel frühestens dann begonnen, wenn der Reaktor eine ausreichende Menge Flüssigkeit enthält, die eine Durchmischung des Reaktorinhalts erlaubt. Nach Erreichen des stationären Zustands weist das Reaktionsgemisch vorzugsweise eine durchschnittliche Verweilzeit im ersten Reaktor im Bereich von 10 Minuten bis 300 Minuten, vorzugsweise 20 Minuten bis 200 Minuten, insbesondere 30 Minuten bis 150 Minuten, auf.

Der zweite Reaktor (und gegebenenfalls weitere Reaktoren) soll der Vervollständigung der Reaktion durch weitere Umsetzung des Reaktionsgemischs bei ausreichender Verweilzeit dienen. Geeignete Reaktoren für diese Reaktionsstufe sind beispielsweise Rührkessel oder Rohrreaktoren. Zur Sicherstellung einer ausreichenden Verweilzeit kann der zweite Reaktor (und falls vorhanden weitere Reaktoren) z. B. teilweise oder vollständig mit einer Packung gefüllt sein und/oder über einen in einem externen Kreislauf geführten Strom (externer Umlaufstrom, Flüssigkeitsumlauf) verfügen. Spezielle Verweilzeitpackungen, welche die Einhaltung der erforderlichen Reaktionszeit ermöglichen, sind dem Fachmann bekannt. In einer speziellen Ausführungsform verfügt der zweite Reaktor über einen in einem externen Kreislauf geführten Strom. Nach Erreichen des stationären Zustands weist das Reaktionsgemisch vorzugsweise eine durchschnittliche Verweilzeit im zweiten Reaktor im Bereich von 10 Minuten bis 300 Minuten, vorzugsweise 20 Minuten bis 200 Minuten, insbesondere 30 Minuten bis 150 Minuten, auf.

Wird die Umsetzung in Schritt b) kontinuierlich in einem Reaktionssystem aus mehreren Reaktoren durchgeführt, so liegt die Temperatur in allen Reaktoren vorzugsweise in einem Bereich von 50 bis 110 °C, besonders bevorzugt von 60 bis 105 °C, insbesondere von 75 bis 100 °C. Umfasst das Reaktionssystem mehr als einen Reaktor, so können diese gleiche oder verschiedene Temperaturen aufweisen. Desgleichen kann ein Reaktor mehrere Reaktionszonen aufweisen, die bei verschiedenen Temperaturen betrieben werden. So kann beispielsweise in einer zweiten Reaktionszone eines einzelnen Reaktors eine höhere Temperatur als in der ersten Reaktionszone oder im zweiten Reaktor einer Reaktorkaskade eine höhere Temperatur als im ersten Reaktor eingestellt werden, z. B. um einen möglichst vollständigen Umsatz zu erzielen.

Wie zuvor ausgeführt, erfolgt die Umsetzung in Schritt b) in einer ersten bevorzugten Ausführungsform bei einem Druck im Bereich von 850 bis 1150 mbar, bevorzugt von 900 bis 1100 mbar. In einer zweiten bevorzugten Ausführungsform erfolgt die Umsetzung in Schritt b) bei einem Druck im Bereich von 100 bis 800 mbar, bevorzugt von 200 bis 700 mbar. Diese Werte gelten prinzipiell unabhängig davon, ob die Umsetzung in Schritt b) diskontinuierlich oder kontinuierlich durchgeführt wird. Diese Werte gelten prinzipiell auch unabhängig davon, ob die Umsetzung in Schritt b) in einem Reaktor oder in mehreren Reaktoren durchgeführt wird. Der Reaktionsdruck kann beim Einsatz mehrerer Reaktoren in allen Reaktoren gleich oder in einzelnen Reaktoren unterschiedlich sein.

In einer bevorzugten Ausführungsform der zuvor beschriebenen Niederdruckvariante wird während der Umsetzung in Schritt b) destillativ Wasser aus dem Reaktionsgemisch entfernt. Dieses destillative Abtrennen von Wasser kann prinzipiell nach üblichen, dem Fachmann bekannten Verfahren erfolgen. In einer geeigneten Ausführungsform wird während der Reaktion ein wasserhaltiges Gas abdestilliert, der Brüden zumindest teilweise kondensiert, und das Kondensat abgetrennt. Zur Kondensation bzw. partiellen Kondensation des Brüdens können alle geeigneten Kondensatoren verwendet werden. Diese können mit beliebigen Kühlmedien gekühlt werden. Geeignet sind beispielsweise Kondensatoren mit Luftkühlung und/oder Wasserkühlung. Die Kondensationswärme des Brüdens kann im erfindungsgemäßen Prozess oder einem anderen Prozess genutzt werden.

Wird die Umsetzung in Schritt b) in mehreren Reaktoren durchgeführt, so kann die destillative Abtrennung von Wasser nur aus einem oder aus mehreren der Reaktoren erfolgen. In einer speziellen Ausführung wird die Umsetzung in Schritt b) in mehreren Reaktoren durchgeführt und erfolgt die destillative Abtrennung von Wasser zumindest aus dem ersten Reaktor in Stromrichtung des Reaktionsgemischs. Spezieller erfolgt die destillative Abtrennung von Wasser nur aus dem ersten Reaktor in Stromrichtung des Reaktionsgemischs.

### Schritt c)

In Schritt c) des erfindungsgemäßen Verfahrens wird dem Umsetzungsprodukt aus Schritt b) wenigstens eine Base zugegeben, wobei sich eine organische Phase bildet, die das Pantolacton (I) enthält, und eine wässrige Phase, die das Basensalz der starken Säure enthält.

In einer bevorzugten Ausgestaltung des erfindungsgemäßen Verfahrens wird in Schritt c) dem Umsetzungsprodukt aus Schritt b) wenigstens eine Base zugegeben, bis der pH-Wert in einem Bereich von 4 bis 6, insbesondere von 4,5 bis 5,5, liegt. Ein besonders bevorzugter pH-Wert liegt bei etwa 5.

Die Bestimmung des pH-Wertes kann nach der eingangs beschriebenen Vorgehensweise erfolgen. Die angegebenen pH-Werte sind temperaturkompensiert und beziehen sich auf 20 °C. Die pH-Wertbestimmung in Schritt c) erfolgt vorzugsweise in der gut durchmischten Emulsion. Es hat sich gezeigt, dass die so ermittelten Messwerte den nach einer Phasentrennung in der wässrigen Phase ermittelten Werten entsprechen.

Erfindungsgemäß wird als Base NH₃ in gasförmiger oder in wässriger Form eingesetzt.

Wird in Schritt c) als Base NH₃ in wässriger Form eingesetzt, so liegt der Massenanteil an NH₃ vorzugsweise in einem Bereich von 15 bis 30 %. Geeignet ist z. B. eine technisch zur Verfügung stehende 25%ige Ammoniaklösung (Konzentration 13,30 mol/l).

Wie zuvor ausgeführt, kann es von Vorteil sein, beim Einsatz wässriger Basen in Schritt c) zuvor während der Umsetzung in Schritt b) destillativ Wasser aus dem Reaktionsgemisch zu entfernen.

Bevorzugt liegt der Wassergehalt des Reaktionsgemischs in Schritt c) nach Zugabe der Base in einem Bereich von 40 bis 60 Gew.-%, bevorzugt von 45 bis 50 Gew.-%, bezogen auf das Gesamtgewicht des Reaktionsgemischs.

### Schritt d)

Das in Schritt c) erhaltene zweiphasige Produkt wird in Schritt d) einer Phasentrennung unterzogen.

Im einfachsten Fall erfolgt die Umsetzung in Schritt c) in batch-Fahrweise. Dann kann die Phasentrennung nach Abschluss der Basenzugabe in demselben Reaktor durchgeführt werden, der für die Umsetzung in Schritt c) eingesetzt wird.

In einer bevorzugten Ausführungsform des erfindungsgemäßen Verfahrens wird das in Schritt c) erhaltene zweiphasige Produkt in Schritt d) einer Phasentrennung in einer separaten Phasentrennvorrichtung unterzogen.

Geeignete Phasentrennvorrichtungen und Verfahren zur Phasentrennung (flüssigflüssig-Trennung) sind beispielsweise in Ullmann's Encyclopedia of Industrial Chemistry, sixth edition, 2000 electronic release, Kapitel "Liquid-Liquid Extraction", dort besonders in Unterkapitel 4 "Phase-Separation Equipment", beschrieben. Bevorzugte Phasentrennvorrichtungen sind Phasenscheider, Dekantierer, Zentrifugen, Coalescer, Mixer-Settler-Apparate und Kombinationen davon. Insbesondere erfolgt die Phasentrennung in Schritt d) unter Verwendung wenigstens eines Phasentrennbehälters (Absetzbehälter).

Bevorzugt lässt man in Schritt d) die Phasen bei einer Temperatur im Bereich von 50 bis 100 °C, besonders bevorzugt von 60 bis 98 °C, insbesondere von 70 bis 95 °C, separieren. Dazu wird vorzugsweise wenigstens eine beheizbare Phasentrennvorrichtung eingesetzt. Aufgrund der besseren Löslichkeit der Basensalze der starken Säure (z. B. von Ammoniumsulfat) in Wasser ist es vorteilhaft, die Phasentrennung bei erhöhter Temperatur durchzuführen, um den Aussalzeffekt möglichst optimal auszunutzen. Somit kann der Anteil an Pantolacton in der wässrigen Phase minimiert werden.

In einer speziellen Ausführungsform werden zur Überführung des in Schritt c) erhaltenen zweiphasigen Produkts in eine Phasentrennvorrichtung wärmeisolierte oder temperierte Leitungen eingesetzt. Somit kann ein unerwünschtes Ausfallen des Basensalzes der starken Säure (z. B. Ammoniumsulfat) wirkungsvoll vermieden werden.

Die in Schritt d) eingesetzte Phasentrennvorrichtung kann über eine Vorrichtung zur Zuführung eines Inertgases erfolgen. Dabei ist es in der Regel ausreichend, wenigstens ein Inertgas in den Gasraum oberhalb der flüssigen Phasen einzuspeisen und an anderer Stelle aus dem Gasraum einen Abgasstrom auszuleiten.

Bevorzugt weist die in Schritt d) erhaltene organische Phase einen Gehalt an Panolacton (I) von wenigstens 65 Gew.-%, besonders bevorzugt von wenigstens 70 Gew.-%, insbesondere von wenigstens 72 Gew.-%, speziell von wenigstens 75 Gew.-%, bezogen auf das Gesamtgewicht der organischen Phase, auf.

Bevorzugt weist die in Schritt d) erhaltene organische Phase einen Gehalt an Wasser von höchstens 25 Gew.-%, besonders bevorzugt von höchstens 20 Gew.-%, insbesondere von höchstens 18 Gew.-%, speziell von höchstens 17 Gew.-%, bezogen auf das Gesamtgewicht der organischen Phase, auf.

Bevorzugt weist die in Schritt d) erhaltene organische Phase im Wesentlichen keine Verseifungsprodukte des Pantolactons (Pantoate) auf. Bevorzugt weist die in Schritt d) erhaltene organische Phase einen Gehalt an Pantoaten von höchstens 1 Gew.-%, besonders bevorzugt von höchstens 0,5 Gew.-%, insbesondere von höchstens 0,1 Gew.-%, speziell von höchstens 0,05 Gew.-%, bezogen auf das Gesamtgewicht der organischen Phase, auf.

Bevorzugt weist die in Schritt d) erhaltene wässrige Phase einen Gehalt an Panolacton (I) von höchstens 5 Gew.-%, besonders bevorzugt von höchstens 4 Gew.-%, insbesondere von höchstens 3 Gew.-%, speziell von höchstens 2 Gew.-%, bezogen auf das Gesamtgewicht der wässrigen Phase, auf.

Bevorzugt weist die in Schritt d) erhaltene wässrige Phase einen Gehalt an dem Basensalz der starken Säure von wenigstens 35 Gew.-%, besonders bevorzugt von wenigstens 40 Gew.-%, insbesondere von wenigstens 43 Gew.-%, speziell von wenigstens 45 Gew.-%, bezogen auf das Gesamtgewicht der wässrigen Phase, auf.

Beispielsweise wird beim Einsatz von Schwefelsäure als starke Säure und von Ammoniak als Base nach dem erfindungsgemäßen Verfahren eine optimale Anreicherung von D,L-Pantolacton in der organischen Phase von etwa 75 Gew.-% bei einem pH Wert um 5 erzielt werden. Damit übereinstimmend sinkt der Rest-Pantolactongehalt in der Wasserphase durch den Aussalzeffekt des entstehenden Ammoniumsulfats auf etwa 1,95 Gew.-%.

### Schritt e)

Eine spezielle Ausführung der Erfindung betrifft ein Verfahren, bei man zusätzlich
e) die organische Phase, die das Pantolacton (I) enthält, einer Aufarbeitung unterzieht.

Zur Aufarbeitung kann die organische Phase wenigstens einem Aufarbeitungsschritt unterzogen werden. Dabei handelt es sich in der Regel um fachübliche Aufarbeitungsverfahren. Die Aufarbeitung in Schritt e) erfolgt vorzugsweise durch Destillation, Waschen, Trocknung, Filtration, Strippung oder eine Kombination aus wenigstens zwei dieser Maßnahmen. Die Aufarbeitungsschritte können jeweils ein- oder mehrfach durchlaufen werden.

Zum Waschen wird die organische Phase, die das Pantolacton (I) enthält, in mindestens einem Waschapparat mit einem wässrigen Waschmedium in Kontakt gebracht. Geeignete Waschmedien sind Wasser und wässrige Salzlösungen. Bevorzugt als Waschmedien sind gesättigte wässrige Salzlösungen des Salzes aus der in Schritt b) eingesetzten starken Säure und der in Schritt c) eingesetzten Base. Somit kann in der Regel z. B. der Wassergehalt der organischen Phase weiter reduziert werden. Die Wäsche kann beispielsweise in einem Rührbehälter oder in anderen herkömmlichen Apparaturen, z. B. in einer Kolonne oder Mixer-Settler-Apparatur, durchgeführt werden.

Eine Filtration kann beispielsweise sinnvoll sein, um ausgefallene Feststoffe, z. B. Salze, zu entfernen.

Bevorzugt wird die organische Phase in Schritt e) einer destillativen Auftrennung unter Erhalt wenigstens einer an Pantolacton (I) angereicherten Fraktion und wenigstens einer an Pantolacton (I) abgereicherten Fraktion unterzogen.

Für die Destillation kann grundsätzlich jede Destillationskolonne verwendet werden. Die Destillation kann einstufig oder mehrstufig durchgeführt werden. Geeignete Vorrichtungen für die destillative Aufarbeitung umfassen Destillationskolonnen, wie Bodenkolonnen, die mit Glocken, Siebplatten, Siebböden, Packungen, Einbauten, Ventilen, Seitenabzügen, etc. versehen sein können. Geeignet sind auch Trennwandkolonnen, die mit Seitenabzügen, Rückführungen, etc. versehen sein können. Zur Destillation kann eine Kombination aus zwei oder mehr als zwei Destillationskolonnen eingesetzt werden. Geeignet sind weiterhin Verdampfer, wie Dünnschichtverdampfer, speziell Fallfilmverdampfer, Dünnschichtverdampfer mit rotierenden Wischern, speziell Sambay-Verdampfer, etc. und Kombinationen davon.

Nach dem erfindungsgemäßen Verfahren wird ein D,L-Enantiomerengemisch von Pantolacton erhalten, das sich vorteilhaft für den Einsatz zur Racematspaltung eignet. Dabei kann man z. B. direkt die organische Phase aus Schritt d) oder ein daraus nach Aufarbeitung erhaltenes Produkt einsetzen. In einer speziellen Ausführung wird zur Racematspaltung eine an Pantolacton angereicherte Fraktion aus dem erfindungsgemäßen Schritt e) eingesetzt.

Die Racematspaltung kann nach üblichen, dem Fachmann bekannten Verfahren erfolgen. So beschreibt z. B. die EP 0443406 A2 die Herstellung von (R)-(-)-Pantolacton durch Spaltung von racemischem Pantolacton. Dabei wird racemisches Pantolacton in Gegenwart von Lipase aus Pseudomonas oder Schweinepankreas einer enantioselektiven Veresterung mit bestimmten Vinylestern oder Methylvinylestern unterzogen, wobei optisch aktives (R)-(-)-Pantolacton zurückbleibt und von dem gebildeten Pantolactonester getrennt wird.

Die WO 0132890 beschreibt L-Pantolacton-Hydrolase und ein Verfahren zur Herstellung von D-Pantolacton.

Bezüglich der Racematspaltung wird auf die Lehre der genannten Dokumente in vollem Umfang Bezug genommen.

### FIGURENBESCHREIBUNG

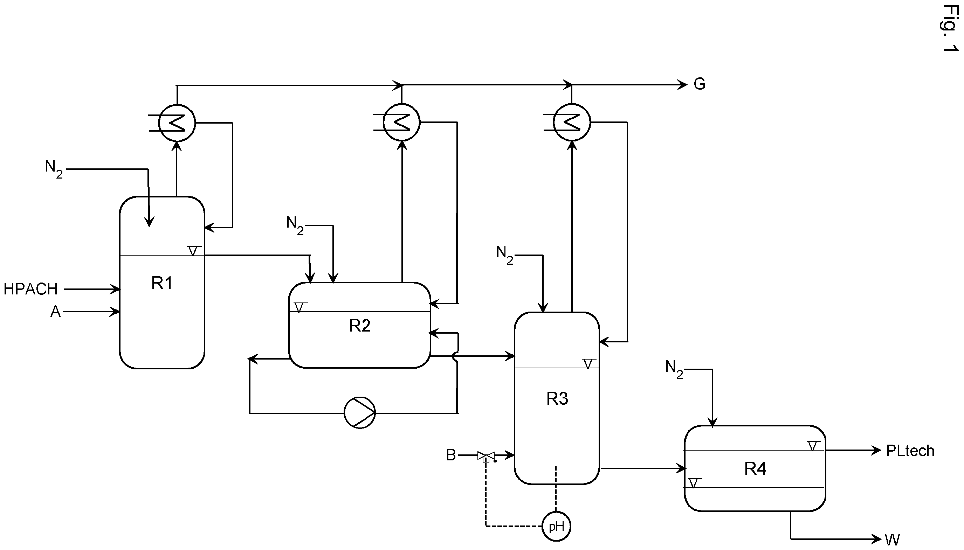

Nachfolgend wird das erfindungsgemäße Verfahren anhand von Figur 1 näher erläutert, ohne es auf diese Ausführung zu beschränken.

In Figur 1 werden folgende Abkürzungen bzw. Bezugszeichen verwendet:
- HPACH: wässrige Hydroxypivalinaldehydcyanhydrinlösung
- A: Säure, beispielsweise 98%ige H₂SO₄
- B: Base, beispielsweise NH₃
- N₂: Inertgasstrom, insbesondere Stickstoff
- G: Abgas
- W: wässrige Phase, Unterphase
- PLtech: organische Phase mit Pantolacton
- R1: Reaktor
- R2: Verweilzeitbehälter
- R3: Neutralisationsbehälter
- R4: Phasentrennbehälter

Figur 1 zeigt schematisch eine Vorrichtung zur kontinuierlichen Durchführung des erfindungsgemäßen Verfahrens. In einen Reaktor R1 werden wässrige Hydroxypivalinaldehydcyanhydrinlösung (HAPACH) und Schwefelsäure (A) parallel, d.h. zeitgleich über getrennte Leitungen, beispielsweise über Pumpen, zudosiert. Die Umsetzung im Reaktor R1 erfolgt unter Inertgasatmosphäre (N₂) homogen in flüssiger Phase. Die einphasige Reaktionsmischung gelangt nach der gewünschten Verweilzeit beispielsweise über einen Überlauf in den temperierten, liegenden Verweilzeitbehälter R2. Zur besseren Durchmischung seines Inhalts ist der Reaktor R2 mit einem externen Kreislauf versehen. Mit ausreichender Verweilzeit zur vollständigen Umsetzung fließt der Reaktionsansatz in den gerührten Neutralisationsbehälter R3. Im Reaktor R3 wird der pH-Wert kontinuierlich oder in bestimmten Zeitabständen erfasst. Durch Zudosierung von gasförmigem Ammoniak wird unter gleichzeitiger Kühlung der gewünschte pH Wert eingestellt. Die resultierende Emulsion wird in den Phasentrennbehälter R4 geleitet. Dort bilden sich eine organische Oberphase, die das Pantolacton (Pltech) enthält, und eine wässrige Unterphase (wässrige Phase). Die beiden Phasen werden getrennt abgezogen. Die das Produkt enthaltende organische Phase kann einer weiteren Aufarbeitung zugeführt werden. Die wässrige Unterphase kann gegebenenfalls nach einer Zwischenreinigung in das Verfahren zurückgeführt oder aus dem Verfahren ausgeschleust werden.

Die folgenden Beispiele dienen der Verdeutlichung der Erfindung, ohne sie in irgendeiner Weise zu beschränken.

### BEISPIELE

### Beispiel 1:

Umsetzung von Hydroxypivalinaldehydcyanhydrin (HAPACH) mit konzentrierter Schwefelsäure und gasförmigem Ammoniak in Semibatchfahrweise bei Normaldruck zu D,L-Pantolacton

Zur Vorlage einer Mischung aus 132,3 g einer ca. 38%igen wässrigen Hydroxypivalinaldehydcyanhydrinlösung und 41,6 g 96%iger Schwefelsäure werden, beginnend bei ca. 80 °C, unter Rühren und Stickstoffüberlagerung 962,8 g HAPACH-Lösung und 303,4 g 96%ige Schwefelsäure innerhalb von 0,5 h parallel zudosiert. Nach beendeter Dosierung lässt man die einphasige Reaktionsmischung (pH = 0,3) 2 h bei 100 °C nachrühren und kühlt anschließend auf 70 °C ab. Bei Zugabe von 62,5 g gasförmigem Ammoniak über Tauchrohr bis zu einem pH Wert von 5,0 erfolgt die Ausbildung zweier Phasen.

Die Phasentrennung bei 80 °C liefert 545,0 g organische Oberphase mit einem Gehalt an D,L-Pantolacton von 75,1 Gew.-% (HPLC) und 948,2 g wässrige Unterphase mit einem D,L-Pantolactonanteil von 1,47 Gew.-% (HPLC).

### Beispiel 2:

Umsetzung von Hydroxypivalinaldehydcyanhydrin (HAPACH) mit konzentrierter Schwefelsäure und 25%igem wässrigen Ammoniak in Batchfahrweise unter Vakuum zu D,L-Pantolacton 972,5 g einer ca. 38%igen wässrigen Hydroxypivalinaldehydcyanhydrinlösung vorlegen und bei einem Druck von 570 bis 580 mbar abs. und einer Temperatur von 80 bis 85 °C 307,8 g 96%ige Schwefelsäure innerhalb von 45 Minuten zudosieren. Die Temperatur steigt auf etwa 90 °C und rund 177,4 g Wasser und Leichtsieder destillieren ab. Nach beendeter Schwefelsäuredosierung wird die einphasige Reaktionsmischung 2 h bei 100 °C nachgerührt und das Vakuum anschließend durch Belüften mit Stickstoff aufgehoben. Zugabe von 233,4 g 25%igem wässrigen Ammoniak bis pH 5,0 führt zur Ausbildung zweier Phasen.

Organische Oberphase: 467,4 g mit einem D,L-Pantolactonanteil von 75,7 Gew.-% (HPLC)
Wässrige Unterphase: 850,6 g mit einem D,L-Pantolactongehalt von 1,36 Gew.-% (HPLC)

### Beispiel 3:

Umsetzung von Hydroxypivalinaldehydcyanhydrin (HAPACH) mit konzentrierter Schwefelsäure und gasförmigem Ammoniak in kontinuierlicher Fahrweise bei Atmosphärendruck zu D,L-Pantolacton

Zur Umsetzung wird eine Vorrichtung gemäß Figur 1 eingesetzt. In den Reaktor R 330 werden unter Überlagerung mit Stickstoff und Rühren bei 94 bis 96 °C aus gewogenen Vorlagen ca. 36%ige wässrige Hydroxypivalaldehydcyanhydrinlösung (HAPACH) mit einer Rate von 835,5 g/h und 96%ige wässrige Schwefelsäure mit einer Rate von 250,0 g/h parallel über Pumpen zudosiert.

Die einphasige Reaktionsmischung gelangt nach einer Verweilzeit von ca. 80 Minuten über einen freien Überlauf in den ebenfalls auf 94 bis 96 °C temperierten, liegenden Verweilzeitbehälter B 331, dessen Inhalt zur besseren Durchmischung mit einem 6-fachen Volumenwechsel pro Stunde über einen externen Kreislauf umgepumpt wird. Der vollständig umgesetzte Reaktionsansatz fließt dann nach einer Verweilzeit von ca. 80 Minuten in den gerührten Neutralisationsbehälter R 643. Durch Zudosierung von gasförmigem Ammoniak mit einer Rate von 46 g/h wird unter gleichzeitiger Kühlung auf 79 bis 80 °C ein pH Wert von 5,0 eingestellt. Aus der resultierenden Emulsion trennen sich im nachfolgenden Behälter B 732 bei 80 °C 379,1 g/h organische Oberphase mit einem Gewichtsanteil von 75,5 % D,L-Pantolacton (bestimmt mittels HPLC) ab. Die wässrige Unterphase wird nach ihrer Entnahme im Ablauf noch mit ca. 235 g/h vollentsalztem Wasser verdünnt, um Kristallisatbildung durch Ammoniumsulfat zu vermeiden.

Die verdünnte wässrige Lösung (Gesamtmenge 986,8 g/h), enthält noch weitere 1,35 Gew.-% D,L-Pantolacton (bestimmt mittels HPLC).

### Beispiel 4:

Umsetzung von Hydroxypivalinaldehydcyanhydrin (HAPACH) mit konzentrierter Schwefelsäure und 25%igem wässrigen Ammoniak in kontinuierlicher Fahrweise unter Vakuum zu D,L-Pantolacton

Zur Durchführung der Reaktion bei vermindertem Druck werden an der Vorrichtung gemäß Figur 1 folgende Veränderungen vorgenommen:
1.) Ersatz des Rückflusskühlers am Reaktor R 330 durch einen Kolonnenkopf mit Destillationsvorlage.
2.) Anschluss des Reaktors R 330 an ein externes Vakuum (ca. 580 mbar) anstelle der Stickstoffüberlagerung.
3.) Ersatz des freien Überlaufs zwischen R 330 und B 331 durch eine Pumpe mit Füllstandsregelung, die den Übergang des Reaktorinhalts aus dem Unterdruckbereich des Reaktors R 330 in die anschließenden Vorrichtungen sicher stellt, die bei Atmosphärendruck betrieben werden.
4.) Verwendung von 25%iger wässriger Ammoniaklösung zur Neutralisation, die aus einer gewogenen Vorlage mittels Pumpe in den Neutralisationsbehälter R 643 gefördert wird.

In den auf 580 mbar evakuierten Reaktor R 330 werden unter Rühren bei 94 bis 96 °C aus gewogenen Vorlagen ca. 38%ige wässrige Hydroxypivalinaldehydcaynhydrinlösung (HAPACH) mit einer Rate von 835,5 g/h und 96%ige wässrige Schwefelsäure mit einer Rate von 250,0 g/h parallel über Pumpen zudosiert, wobei gleichzeitig rund 127 g Wasser und Leichtsieder pro Stunde über den Kolonnenkopf in die Destillationsvorlage abgezogen werden.

Die einphasige Reaktionsmischung wird nach einer Verweilzeit von ca. 45 Minuten unter Füllstandsregelung mit Hilfe einer Pumpe in den Verweilzeitbehälter B 331 gefördert. Der unter Stickstoffüberlagerung auf Atmosphärendruck gehaltenen Verweilzeitbehälter B 331 ist ebenfalls auf 94 bis 96 °C temperiert und der Inhalt wird zur besseren Durchmischung mit einem 6-fachen Volumenwechsel pro Stunde über einen externen Kreislauf umgepumpt.

Der vollständig umgesetzte Reaktionsansatz fließt dann nach einer Verweilzeit von ca. 90 Minuten in den gerührten Neutralisationsbehälter R 643. Durch Zudosierung von 25%igem wässrigen Ammoniak mit einer Rate von 171,2 g/h wird unter gleichzeitiger Kühlung auf 79 bis 80 °C ein pH Wert von 5,0 eingestellt. Aus der resultierenden Emulsion trennen sich im nachfolgenden Behälter B 732 bei 80 °C 374,6 g/h organische Oberphase mit einem Gewichtsanteil von 76,15 % D,L-Pantolacton (bestimmt mittels HPLC) ab. Die wässrige Unterphase wird nach ihrer Entnahme im Ablauf noch mit ca. 180 g/h vollentsalztem Wasser verdünnt, um Kristallisatbildung durch Ammoniumsulfat zu vermeiden. Die verdünnte wässrige Lösung (Gesamtmenge 936,2 g/h), enthält noch weitere 1,28 Gew.-% D,L-Pantolacton (bestimmt mittels HPLC).

## Patentansprüche

1. Verfahren zur Herstellung von Pantolacton (I) 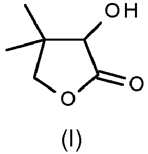 bei dem man
a) eine wässrige Zusammensetzung, die Hydroxypivalaldehydcyanhydrin (II) 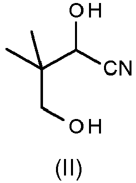 enthält, als Ausgangsmaterial bereitstellt,
b) das in Schritt a) bereitgestellte Ausgangsmaterial einer Umsetzung mit H₂SO₄ als Säure bei einem pH-Wert von kleiner als 1,1 in einer homogenen flüssigen Phase unterzieht,
c) dem Umsetzungsprodukt aus Schritt b)NH₃ in gasförmiger oder wässriger Form als Base zugegeben wird, bis der pH-Wert in einem Bereich von 3 bis 7 liegt und wobei der Wassergehalt des Reaktionsgemischs in Schritt c) nach Zugabe der Base in einem Bereich von 40 bis 60 Gew.-%, bezogen auf das Gesamtgewicht des Reaktionsgemischs, liegt, wobei sich eine organische Phase bildet, die das Pantolacton (I) enthält, und eine wässrige Phase, die das Basensalz der starken Säure enthält,
d) die Phasen separieren lässt und die organische Phase von der wässrigen Phase abtrennt.

2. Verfahren nach Anspruch 1, wobei man zur Bereitstellung des Ausgangsmaterials in Schritt a):
a1) Formaldehyd mit Isobutyraldehyd einer Aldoladdition unter Erhalt von Hydroxypivalaldehyd unterzieht, und
a2) den Hydroxypivalaldehyd mit einer Cyanidquelle unter Erhalt von Hydroxypivalaldehydcyanhydrin (II) umsetzt.

3. Verfahren nach einem der vorhergehenden Ansprüche, wobei als Säure in Schritt b) wenigstens 90%ige H₂SO₄, eingesetzt wird.

4. Verfahren nach einem der vorhergehenden Ansprüche, wobei während der Umsetzung in Schritt b) der pH-Wert des Reaktionsgemischs in einem Bereich von 0,2 bis 1 gehalten wird.

5. Verfahren nach einem der vorhergehenden Ansprüche, wobei in Schritt b) das Molverhältnis der eingesetzten Säure zu Hydroxypivalaldehydcyanhydrin (II) in einem Bereich von 0,5 : 1 bis 2 : 1 liegt.

6. Verfahren nach einem der vorhergehenden Ansprüche, wobei die Umsetzung in Schritt b) bei einer Temperatur von 50 bis 110 °C erfolgt.

7. Verfahren nach einem der Ansprüche 1 bis 6, wobei die Umsetzung in Schritt b) bei einem Druck im Bereich von 850 bis 1150 mbar erfolgt.

8. Verfahren nach einem der Ansprüche 1 bis 6, wobei die Umsetzung in Schritt b) bei einem Druck im Bereich von 100 bis 800 mbar erfolgt.

9. Verfahren nach Anspruch 8, wobei während der Umsetzung in Schritt b) destillativ Wasser aus dem Reaktionsgemisch entfernt wird.

10. Verfahren nach einem der vorhergehenden Ansprüche, wobei in Schritt c) dem Umsetzungsprodukt aus Schritt b) wenigstens eine Base zugegeben wird, bis der pH-Wert in einem Bereich von 4 bis 6 liegt.

11. Verfahren nach einem der vorhergehenden Ansprüche, wobei die Umsetzung in Schritt b) bei einem Druck im Bereich von 100 bis 800 mbar erfolgt und während der Umsetzung in Schritt b) destillativ Wasser aus dem Reaktionsgemisch entfernt wird und in Schritt c) dem Reaktionsprodukt aus Schritt b) wässriges NH₃ als Base zugegeben wird.

12. Verfahren nach einem der vorhergehenden Ansprüche, wobei der Wassergehalt des Reaktionsgemischs in Schritt c) nach Zugabe der Base in einem Bereich von 45 bis 50 Gew.-%, bezogen auf das Gesamtgewicht des Reaktionsgemischs, liegt.

13. Verfahren nach einem der vorhergehenden Ansprüche, wobei man in Schritt d) die Phasen bei einer Temperatur im Bereich von 50 bis 100 °C separieren lässt.

14. Verfahren nach einem der vorhergehenden Ansprüche, wobei man zusätzlich
e) die organische Phase, die das Pantolacton (I) enthält, einer Aufarbeitung unterzieht.

15. Verfahren nach Anspruch 14, wobei man die organische Phase einer destillativen Auftrennung unter Erhalt wenigstens einer an Pantolacton (I) angereicherten Fraktion und wenigstens einer an Pantolacton (I) abgereicherten Fraktion unterzieht.

16. Verfahren nach Anspruch 14 oder 15, wobei man die organische Phase oder wenigstens eine an Pantolacton (I) angereicherte Fraktion einer Racematspaltung unter Erhalt wenigstens einer an D-Pantolacton angereicherten Fraktion unterzieht.

## Claims

1. A process for the preparation of pantolactone (I) 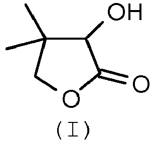 in which
a) an aqueous composition which comprises hydroxypivalaldehyde cyanohydrin (II) 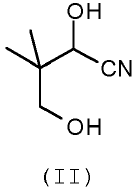 is provided as starting material,
b) the starting material provided in step a) is subjected to a reaction with H₂SO₄ as acid at a pH of less than 1.1 in a homogeneous liquid phase,
c) NH3 in gaseous or aqueous form is added as base to the reaction product from step b) until the pH is in a range from 3 to 7 and with the water content of the reaction mixture in step c) after the addition of the base being in a range from 40 to 60% by weight, based on the total weight of the reaction mixture, with an organic phase being formed which comprises the pantolactone (I), and an aqueous phase which comprises the base salt of the strong acid,
d) the phases are left to separate and the organic phase is separated off from the aqueous phase.

2. The process according to claim 1, where, for the provision of the starting material in step a):
a1) formaldehyde is subjected with isobutyraldehyde to an aldol addition, giving hydroxypivalaldehyde, and
a2) the hydroxypivalaldehyde is reacted with a cyanide source, giving hydroxypivalaldehyde cyanohydrin (II).

3. The process according to any one of the preceding claims, where the acid used in step b) is at least 90% strength H₂SO₄.

4. The process according to any one of the preceding claims, where, during the reaction in step b), the pH of the reaction mixture is kept in a range from 0.2 to 1.

5. The process according to any one of the preceding claims, where, in step b), the molar ratio of the acid used to hydroxypivalaldehyde cyanohydrin (II) is in a range from 0.5 : 1 to 2 : 1.

6. The process according to any one of the preceding claims, where the reaction in step b) takes place at a temperature from 50 to 110°C.

7. The process according to any one of claims 1 to 6, where the reaction in step b) takes place at a pressure in the range from 850 to 1150 mbar.

8. The process according to any one of claims 1 to 6, where the reaction in step b) takes place at a pressure in the range from 100 to 800 mbar.

9. The process according to claim 8, where, during the reaction in step b), water is removed distillatively from the reaction mixture.

10. The process according to any one of the preceding claims, where, in step c), at least one base is added to the reaction product from step b) until the pH is in a range from 4 to 6.

11. The process according to any one of the preceding claims, where the reaction in step b) takes place at a pressure in the range from 100 to 800 mbar, and during the reaction in step b) water is removed distillatively from the reaction mixture and, in step c), aqueous NH₃ is added as base to the reaction product from step b).

12. The process according to any one of the preceding claims, where the water content of the reaction mixture in step c) following the addition of the base is in a range from 45 to 50% by weight, based on the total weight of the reaction mixture.

13. The process according to any one of the preceding claims, where, in step d), the phases are left to separate at a temperature in the range from 50 to 100°C.

14. The process according to any one of the preceding claims, where, additionally,
e) the organic phase which comprises the pantolactone (I) is subjected to a work-up.

15. The process according to claim 14, where the organic phase is subjected to a distillative separation, giving at least one fraction enriched in pantolactone (I) and at least one fraction depleted in pantolactone (I).

16. The process according to claim 14 or 15, where the organic phase or at least one fraction enriched in pantolactone (I) is subjected to a racemate cleavage, giving at least one fraction enriched in D-pantolactone.

## Revendications

1. Procédé de fabrication de pantolactone (I) 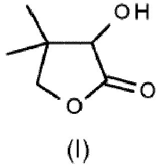 selon lequel
a) une composition aqueuse, qui contient de l'hydroxypivalaldéhyde-cyanhydrine (II) 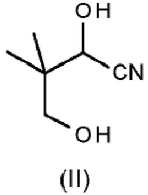 est préparée en tant que matériau de départ,
b) le matériau de départ préparé à l'étape a) est soumis à une réaction avec H₂SO₄ en tant qu'acide à un pH inférieur à 1,1 dans une phase liquide homogène,
c) du NH₃ sous forme gazeuse ou aqueuse est ajouté en tant que base au produit de réaction de l'étape b), jusqu'à ce que le pH se situe dans une plage allant de 3 à 7, la teneur en eau du mélange réactionnel à l'étape c) après l'ajout de la base se situant dans une plage allant de 40 à 60 % en poids, par rapport au poids total du mélange réactionnel, une phase organique se formant, qui contient la pantolactone (I), et une phase aqueuse, qui contient le sel basique de l'acide fort,
d) les phases sont laissées se séparer, et la phase organique est séparée de la phase aqueuse.

2. Procédé selon la revendication 1, selon lequel, pour la préparation du matériau de départ à l'étape a) :
a1) du formaldéhyde est soumis avec de l'isobutyraldéhyde à une addition d'aldolisation pour obtenir de l'hydroxypivalaldéhyde, et
a2) l'hydroxypivalaldéhyde est mis en réaction avec une source de cyanure pour obtenir l'hydroxypivalaldéhyde-cyanhydrine (II).

3. Procédé selon l'une quelconque des revendications précédentes, selon lequel du H₂SO₄ au moins à 90 % est utilisé en tant qu'acide à l'étape b).

4. Procédé selon l'une quelconque des revendications précédentes, selon lequel le pH du mélange réactionnel est maintenu dans une plage allant de 0,2 à 1 pendant la réaction à l'étape b).

5. Procédé selon l'une quelconque des revendications précédentes, selon lequel le rapport molaire entre l'acide utilisé et l'hydroxypivalaldéhyde-cyanhydrine (II) à l'étape b) se situe dans une plage allant de 0, 5 : 1 à 2 : 1.

6. Procédé selon l'une quelconque des revendications précédentes, selon lequel la réaction à l'étape b) a lieu à une température de 50 à 110 °C.

7. Procédé selon l'une quelconque des revendications 1 à 6, selon lequel la réaction à l'étape b) a lieu à une pression dans la plage allant de 850 à 1 150 mbar.

8. Procédé selon l'une quelconque des revendications 1 à 6, selon lequel la réaction à l'étape b) a lieu à une pression dans la plage allant de 100 à 800 mbar.

9. Procédé selon la revendication 8, selon lequel de l'eau est éliminée du mélange réactionnel par distillation pendant la réaction à l'étape b).

10. Procédé selon l'une quelconque des revendications précédentes, selon lequel au moins une base est ajoutée au produit de réaction de l'étape b) à l'étape c), jusqu'à ce que le pH se situe dans une plage allant de 4 à 6.

11. Procédé selon l'une quelconque des revendications précédentes, selon lequel la réaction à l'étape b) a lieu à une pression dans la plage allant de 100 à 800 mbar, et de l'eau est éliminée du mélange réactionnel par distillation pendant la réaction à l'étape b), et du NH₃ aqueux est ajouté en tant que base au produit de réaction de l'étape b) à l'étape c).

12. Procédé selon l'une quelconque des revendications précédentes, selon lequel la teneur en eau du mélange réactionnel à l'étape c) après l'ajout de la base se situe dans une plage allant de 45 à 50 % en poids, par rapport au poids total du mélange réactionnel.

13. Procédé selon l'une quelconque des revendications précédentes, selon lequel les phases sont laissées se séparer à l'étape d) à une température dans la plage allant de 50 à 100 °C.

14. Procédé selon l'une quelconque des revendications précédentes, selon lequel en outre
e) la phase organique qui contient la pantolactone (I) est soumise à un traitement.

15. Procédé selon la revendication 14, selon lequel la phase organique est soumise à une séparation par distillation pour obtenir au moins une fraction enrichie en pantolactone (I) et au moins une fraction appauvrie en pantolactone (I).

16. Procédé selon la revendication 14 ou 15, selon lequel la phase organique ou au moins une fraction enrichie en pantolactone (I) est soumise à un clivage du racémat pour obtenir au moins une fraction enrichie en D-pantolactone.
